# EUROPEAN PATENT APPLICATION

(11) **EP 3 269 821 A1**
(43) Date of publication of application: **17.01.2018**
(21) Application number: 15884710.3
(22) Date of filing: 18.12.2015
(51) Int. Cl.: C12P 21/06, C07K 1/12, C07K 1/22, C07K 16/00

(54) **METHOD FOR OBTAINING PEPTIDE FRAGMENT FROM ANTIBODY BY MEANS OF PROTEASE DECOMPOSITION REACTION WITH RESTRICTED REACTION FIELD**

(30) Priority: 10.03.2015 JP 2015047740
(71) Applicant: Shimadzu Corporation, Kyoto-shi, Kyoto 604-8511 (JP)
(72) Inventor: SHIMADA Takashi, Kyoto-shi Kyoto 604-8511 (JP); IWAMOTO Noriko, Kyoto-shi Kyoto 604-8511 (JP)
(74) Representative: Webster, Jeremy Mark
(86) International application number: PCT/JP2015/085458
(87) International publication number: WO 2016/143226

(57) **Abstract**

The present invention provides: a method for increasing a yield, from a target monoclonal antibody, of a peptide fragment that comprises an Fab region or a variable region of the antibody or a portion of the region, the method being characterized by including a process in which digestion of the target monoclonal body is performed by bringing the target monoclonal antibody, which is immobilized on inner surfaces of pores of a porous body, and a protease, which is immobilized on surfaces of nanoparticles having an average particle size larger than an average pore diameter of the porous body, into contact with each other in a liquid by stirring or tapping rotation stirring at a low speed of less than 100 rpm so as to maintain a uniform dispersion, the average pore diameter of the porous body being in a range of 10 nm - 200 nm and the average particle size of the nanoparticles being in a range of 50 nm - 500 nm; and a kit for use in the method.

SEQUENCE LISTING

<110>  Shimadzu Corporation

<120>  Method for obtaining peptide fragments from antibody
       by proteolysis in restricted reaction field

<130>  PH-6400-PCT

<150>  JP 2015-047740
<151>  2015-03-10

<160>  10

<170>  PatentIn version 3.5

<210>  1
<211>  9
<212>  PRT
<213>  Artificial

<220>
<223>  antibody fragment

<400>  1

Ile Tyr Pro Thr Asn Gly Tyr Thr Arg
1               5


<210>  2
<211>  9
<212>  PRT
<213>  Artificial

<220>
<223>  antibody fragment

<400>  2

Phe Thr Ile Ser Ala Asp Thr Ser Lys
1               5


<210>  3
<211>  18
<212>  PRT
<213>  Artificial

<220>
<223>  antibody fragment

<400>  3

Ala Ser Gln Asp Val Asn Thr Ala Val Ala Trp Tyr Gln Gln Lys Pro
1               5                   10                  15


Gly Lys



<210>  4

<211>  11
<212>  PRT
<213>  Artificial

<220>
<223>  antibody fragment

<400>  4

Asn Thr Ala Tyr Leu Gln Met Asn Ser Leu Arg
1               5                   10


<210>  5
<211>  7
<212>  PRT
<213>  Artificial

<220>
<223>  antibody fragment

<400>  5

Gly Leu Glu Trp Val Ala Arg
1               5


<210>  6
<211>  8
<212>  PRT
<213>  Artificial

<220>
<223>  antibody fragment

<400>  6

Asp Thr Tyr Ile His Trp Val Arg
1               5


<210>  7
<211>  11
<212>  PRT
<213>  Artificial

<220>
<223>  antibody fragment

<400>  7

Ser Thr Ala Tyr Leu Gln Met Asn Ser Leu Arg
1               5                   10


<210>  8
<211>  9
<212>  PRT
<213>  Artificial

<220>
<223>  antibody fragment

<400>  8

Phe Thr Phe Ser Leu Asp Thr Ser Lys
1               5

<210>  9
<211>  23
<212>  PRT
<213>  Artificial

<220>
<223>  antibody fragment

<400>  9

Gly Leu Glu Trp Val Gly Trp Ile Asn Thr Tyr Thr Gly Glu Pro Thr
1               5                   10                  15

Tyr Ala Ala Asp Phe Lys Arg
            20

<210>  10
<211>  16
<212>  PRT
<213>  Artificial

<220>
<223>  antibody fragment

<400>  10

Val Leu Ile Tyr Phe Thr Ser Ser Leu His Ser Gly Val Pro Ser Arg
1               5                   10                  15

## Description

### TECHNICAL FIELD

The present invention relates to a method for obtaining a peptide fragment, preferably with an increased yield, comprising a specific sequence, from a target monoclonal antibody by a protease decomposition reaction with a restricted reaction field for detection of the monoclonal antibody by mass spectrometry.

### BACKGROUND ART

Along with expansion of treatment using antibody drugs such as trastuzumab (trade name: Herceptin) at medical sites, it has become known that an antibody concentration in blood of a patient has a significant correlation with a survival rate of the patient, and accurately quantifying an antibody concentration in blood has been demanded. Further, also in quality control such as confirmation of pharmacokinetics, or in confirmation of identicalness with original drugs in clinical trials or the like of generic drugs, there are increasing needs in antibody drug identification and in quantification of an antibody drug concentration in blood.

As protein quantification methods, in addition to an enzyme antibody measurement method, a fluorescent antibody measurement method, a radioimmunoassay method, and the like, a method using a liquid chromatography-tandem mass spectrometry (LCMS) apparatus, and the like, are also known (for example, Patent Literature 1, Patent Literature 2, and the like). In the LCMS method, a peptide fragment generated after digestion of a target protein using protease is quantitated by combining an LC method and an MS method. Patent Literature 1 discloses that, in a method for determining an amount of an antibody in a biological sample, after protease digestion of a non-immunoglobulin protein in a sample, an antibody fragment comprising at least a CDR region is generated by a protease treatment using pepsin and is quantified using an LCMS method.

Digestion efficiency of protease digestion is different depending on a kind of a substrate protein to be digested. Therefore, in order to simplify preparation of a peptide fragment sample for mass spectrometry, improvement in digestion efficiency is important. In recent years, as a method for improving efficiency of protease digestion, a method of performing protease digestion in a microenvironment (microreactor) such as nanoparticles has attracted attention. For example, Non-Patent Literature 1 reports an example in which efficiency of trypsin digestion of albumin is improved by immobilizing trypsin in pores of a porous membrane of nylon and passing an albumin solution through the porous membrane. Further, Non-Patent Literature 2 reports that, a protein having a small molecular weight can be selectively trypsin-digested by using mesoporous silica in which trypsin is immobilized in pores. In all of these methods, a protease is immobilized in pores of a porous body, and the immobilized protease and a substrate protein in a liquid phase are caused to react with each other. Such methods are well known and have an advantage that solid-liquid separation, that is, separation of a protein decomposition product and an immobilized protease can be easily performed.

Further, a method for causing an immobilized protease and an immobilized protein to react with each other is also provided. For example, Non-Patent Literature 3 discloses a method for producing an antibody decomposition product containing a CDR peptide or a part of a CDR peptide using a protease immobilized on nanoparticles having a particle size of about 190 nm ± 20 nm by digesting an antibody noncovalently bound via Fc to a protein G bound to surfaces of fine pores having a pore diameter of 50 - 100 nm. This method is a novel method developed and proposed by the present inventors.

### CITATION LIST

### PATENT LITERATURE

Patent Literature 1: Japanese Translation of PCT International Application Publication No. 2010-515020.
Patent Literature 2: Japanese Patent Laid-Open Publication No. 2012-197258.

### NON-PATENT LITERATURE

Non-Patent Literature 1: Fei Xu et. al., Analytical Chemistry, 2010, 82: 10045-10051.
Non-Patent Literature 2: Qianhao Min et. al., Chemical Communications, 2010, 46: 6144-6146.
Non-Patent Literature 3: Noriko Iwamoto et. al., Analyst, 2014, 139: 576-580.

### SUMMARY OF THE INVENTION

### TECHNICAL PROBLEM

When a solid phase substrate and a solid phase enzyme are caused to react with each other as described in the above Non-Patent Literature 3, although the reaction occurs in a liquid, the solid phase substances each have a sedimentation property due to gravity, and thus, it is necessary to maintain dispersibility by stirring or the like.

### SOLUTION TO PROBLEM

The present inventors found that, in a reaction between a solid phase substrate (enzyme protein) and a solid phase enzyme (protease), when specific contact between the solid phases is optimized, a yield of a target decomposition product is significantly increased, and thus accomplished the present invention.

In summary, the present invention includes the following aspects.

(1) A method for obtaining, from a target monoclonal antibody, a peptide fragment comprising an Fab region or a variable region of the antibody or a portion of the region, includes a process in which the target monoclonal antibody is digested with a protease by bringing the target monoclonal antibody, which is immobilized on inner surfaces of pores of a porous body, and the protease, which is immobilized on surfaces of nanoparticles having an average particle size larger than an average pore diameter of the porous body, into contact with each other in a liquid by stirring or tapping rotation stirring at a suitable speed so as to maintain a uniform dispersion.
(2) A method for increasing a yield, from a target monoclonal antibody, of a peptide fragment that comprises an Fab region or a variable region of the antibody or a portion of the region, includes a process in which the target monoclonal body is digested with a protease by bringing the target monoclonal antibody, which is immobilized on inner surfaces of pores of a porous body, and the protease, which is immobilized on surfaces of nanoparticles having an average particle size larger than an average pore diameter of the porous body, into contact with each other in a liquid by stirring or tapping rotation stirring at a low speed of less than 100 rpm so as to maintain a uniform dispersion, the average pore diameter of the porous body being in a range of 10 nm - 200 nm and the average particle size of the nanoparticles being in a range of 50 nm - 500 nm.
(3) In the method described in any one of the aspects (1) and (2), the average particle size of the nanoparticles is 1.5 times to 4 times larger than the average pore diameter of the porous body.
(4) In the method described in any one of the aspects (1) - (3), the stirring or the tapping rotation stirring is performed at a low speed of 50 rpm or less.
(5) In the method described in any one of the aspects (1) - (4), the protease is selected from a group consisting of trypsin, Lys-C, Arg-C, Asp-N, chymotrypsin, V8 protease, and combinations of two or more thereof.
(6) In the method described in any one of the aspects (1) - (5), the target monoclonal antibody is bound to the inner surfaces of the pores of the porous body via a linker molecule.
(7) In the method described in any one of the aspects (1) - (6), the peptide fragment is a peptide fragment that comprises a complementarity-determining region (CDR) or a portion thereof.
(8) The method described in any one of the aspects (1) - (7) further includes a process of separating a target monoclonal antibody or an antibody population containing the target monoclonal antibody from a biological sample.
(9) In the method described in the aspect (8), the biological sample is blood, serum, plasma, a tissue or a cell.
(10) The method described in any one of the aspects (8) and (9) further includes a process of binding the antibody population containing the target monoclonal antibody to the surfaces of the pores of the porous body.
(11) In the method described in any one of the aspects (1) - (10), the liquid is a salt-free buffer solution.
(12) In the method described in any one of the aspects (1) - (11), the target monoclonal antibody is an antibody therapeutic agent.
(13) A kit for use in the method described in any one of the aspects (1) - (12), includes: (i) a porous body having pores capable of immobilizing a target monoclonal antibody, (ii) nanoparticles immobilizing a protease on surfaces thereof, and (iii) an instruction manual for instructing a user to bring the target monoclonal antibody and the protease into contact with each other in a liquid by stirring or tapping rotation stirring at a low speed of less than 100 rpm so as to maintain a uniform dispersion, an average particle size of the nanoparticles being larger than a pore diameter of the porous body.
(14) The kit described in the aspect (13) further includes an affinity means for separating and purifying an antibody.
(12) In the kit described in any one of the aspects (13) and (14), the porous body has a linker molecule bound to the surfaces of the nanoparticles and inner surfaces of the pores.
(16) In the kit described in the aspect (15), the linker molecule is an antibody binding polypeptide.

The method of the present invention provides a method for increasing a yield of a protease digestion product of a target monoclonal antibody as a pretreatment for accurately measuring a concentration of an antibody drug in a biological sample such as blood using an LCMS method, and thus has advantages of leading to protocol optimization for products of analysis kits for antibody drugs and allowing analysis reproducibility to be maintained.

The present specification incorporates a disclosure content of Japanese Patent Application No. 2015-047740 on which priority of the present application is based.

### BRIEF DESCRIPTION OF DRAWINGS

[Fig. 1] This figure is a conceptual diagram for describing a principle of protease digestion with a restricted reaction field in the present invention.
[Fig. 2] This figure is an electropherogram of protease digestion in a test for determining an optimal quantitative ratio of a substrate: protease.
[Fig. 3] This figure is a mass spectrometry (MALDI-TOFMS) spectrum of a digestion time examination experiment.

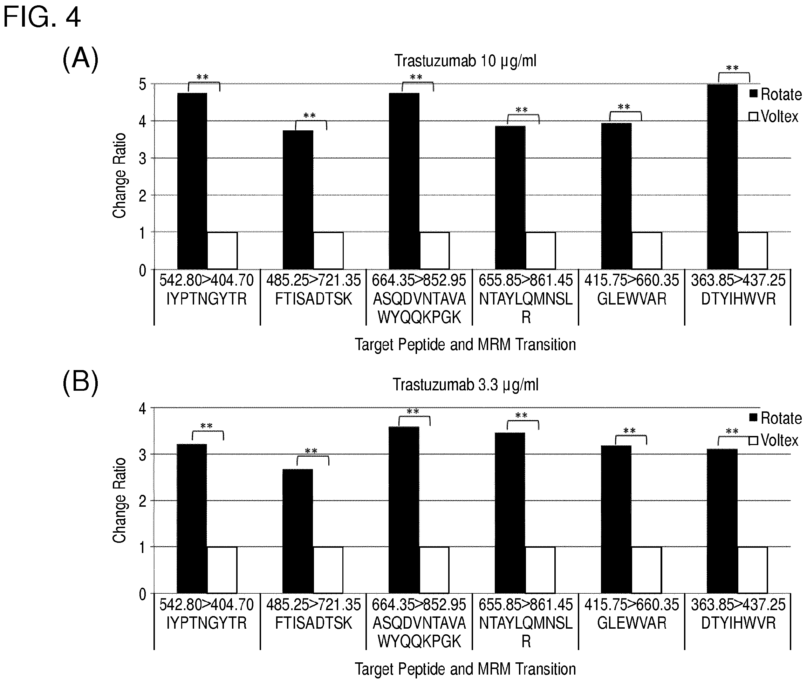

[Fig. 4] This figure is a comparison of peptide yields by trypsin digestion of a trastuzumab antibody in a protease reaction under tapping rotation stirring (rotation speed: 1 - 5 rpm; tapping speed: 2 per minute) or vortex stirring (rotation speed: 100 rpm). In the figure, a black bar indicates a result of tapping rotation stirring ("Rotate") and a white bar indicates a result of vortex stirring ("Voltex"). (A) and (B) respectively illustrate results when an immobilized antibody reacted with 3 µg of an immobilized trypsin at antibody concentrations of 10 µg/ml and 3.3 µg/ml (amounts of the antibody were respectively 1 µg and 0.33 µg in the reactions). A horizontal axis indicates a target peptide and MRM transition. A vertical axis represents a relative value (change ratio) of a peptide yield under tapping rotation stirring when a peptide yield under vortex stirring is 1. MRM is an abbreviation for Multiple Reaction Monitoring, and is a method in which a specific ion (precursor ion) is selected in Q1 (first stage MS) with respect to various ions ionized by an ionization probe, the ion is broken (collision induced dissociation) in a collision cell (q2, collision chamber), and a specific ion is detected from broken ions (product ions) in Q3 (second stage MS). Numbers of MRM transition indicate that a Q1 value (precursor) > a Q3 value (product). Measured peptide fragments are IYPTNGYTR (SEQ ID No: 1), FTISADTSK (SEQ ID No: 2), ASQDVNTAVAWYQQKPGK (SEQ ID No: 3), NTAYLQMNSLR (SEQ ID No: 4), GLEWVAR (SEQ ID No: 5), and DTYIHWVR (SEQ ID No: 6). "**" indicates that p > 0.01.

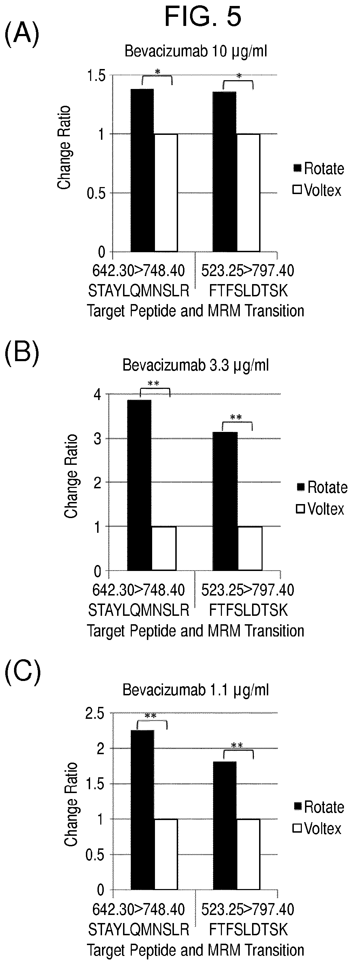

[Fig. 5] This figure is a comparison of peptide fragment yields by trypsin digestion of a bevacizumab antibody under tapping rotation stirring (rotation speed: 1 - 5 rpm; tapping speed: 2 per minute) or vortex stirring (rotation speed: 100 rpm). In the figure, a black bar indicates a result of tapping rotation stirring ("Rotate") and a white bar indicates a result of vortex stirring ("Voltex"). (A), (B) and (C) respectively illustrate results when an immobilized antibody reacted with 3 µg of an immobilized trypsin at antibody concentrations of 10 µg/ml, 3.3 µg/ml and 1.1 µg/ml (amounts of the antibody were respectively 1 µg, 0.33 µg and 0.11 µg in the reactions). A horizontal axis indicates a target peptide and MRM transition. A vertical axis represents a relative value (change ratio) of a peptide yield under tapping rotation stirring when a peptide yield under vortex stirring is 1. Numbers of MRM transition indicate that a Q1 value (precursor) > a Q3 value (product). Measured peptides are STAYLQMNSLR (SEQ ID No: 7), and FTFSLDTSK (SEQ ID No: 8). "**" indicates that p > 0.01.

### DESCRIPTION OF EMBODIMENTS

The present invention is further described in detail.

### 1. Method for Acquiring and Method for Increasing Yield of Protease Digestion Product (Peptide Fragment)

According to a first aspect of the present invention, a method is provided for obtaining, from a target monoclonal antibody, a peptide fragment comprising an Fab region or a variable region of the antibody or a portion of the region. The method includes a process in which the target monoclonal antibody is digested with a protease by bringing the target monoclonal antibody, which is immobilized on inner surfaces of pores of a porous body, and the protease, which is immobilized on surfaces of nanoparticles having an average particle size larger than an average pore diameter of the porous body, into contact with each other in a liquid by stirring or tapping rotation stirring at a suitable speed so as to maintain a uniform dispersion.

According to a second aspect of the present invention, a method is provided for increasing a yield, from a target monoclonal antibody, of a peptide fragment that comprises an Fab region or a variable region of the antibody or a portion of the region. The method includes a process in which the target monoclonal body is digested with a protease by bringing the target monoclonal antibody, which is immobilized on inner surfaces of pores of a porous body, and the protease, which is immobilized on surfaces of nanoparticles having an average particle size larger than an average pore diameter of the porous body, into contact with each other in a liquid by stirring or tapping rotation stirring at a low speed of less than 100 rpm so as to maintain a uniform dispersion, the average pore diameter of the porous body being in a range of 10 nm - 200 nm and the average particle size of the nanoparticles being in a range of 50 nm - 500 nm.

### <Target Antibody>

A measurement target in the method of the present invention is a monoclonal antibody, and is preferably an antibody therapeutic agent (also referred to as an "antibody drug").

In addition to the monoclonal antibody defined below, the term "monoclonal antibody" used in the present specification also includes complexes with additional functions while maintaining specificity of an antibody, for example, an antibody fusion protein (such as etanercept), an antibody-drug complex (such as trastuzumab-emtansin), and the like.

A monoclonal antibody is an immunoglobulin (IgG) and is a biopolymer having a structure in which two heavy chains (each having a molecular weight of about 50 kDa) and two light chains (each having a molecular weight of about 25 kDa) are connected by multiple disulfide bonds. An Fab domain and an Fc domain are connected via a hinge, and the heavy chains and light chains are each formed of a constant region and a variable region. The constant region has a structure that maintains a characteristic Y shape of an antibody, and has an amino acid sequence that is common to most of antibodies derived from the same specie. On the other hand, each variable region has three sites each having a specific sequence called a complementarity-determining region (CDR). A three-dimensional structure defined by the CDR (CDR1, CDR2, CDR3) regions is involved in specific binding with an antigen, and thereby, an antibody-antigen complex is formed. A framework 1 (FR1), a framework 2 (FR2), a framework 3 (FR3) and a framework 4 (FR4) respectively exist before the CDR1, between the CDR1 and the CDR2, between the CDR2 and the CDR3, and after the CDR3, of the variable region of each of the heavy chains and the light chains.

A further characteristic feature of a higher order structure of an antibody is a very flexible hinge region with respect to a constant region having a rigid structure. It is known that there is a site where a specific protein called Protein A or Protein G binds to a C terminal side of a heavy chain (in particular, a region from CH2 to CH3 of an Fc region).

In recent years, a large number of monoclonal antibodies have been developed as antibody drugs that can specifically act on various diseases, and some of them are used in clinical practice. The present invention is a useful method for accurate detection and quantification of a target monoclonal antibody in a biological sample using LCMS analysis. A monoclonal antibody that can be a measurement target in this method is not limited. However, examples of the monoclonal antibody include: human antibodies such as panitumumab, offatumumab, golimumab, and ipilimumab; humanized antibodies such as tocilizumab, trastuzumab, trastuzumab-DM1, bevacizumab, omalizumab, mepolizumab, gemtuzumab, palivizumab, ranibizumab, certolizumab, ocrelizumab, mogamulizumab, and eculizumab; chimeric antibodies such as rituximab, cetuximab, infliximab, and basiliximab; and the like. A monoclonal antibody has a molecular diameter of about 14.5 nm.

According to the method of the present invention, a yield of a peptide fragment can be increased by regioselectively cutting out, by protease digestion, a variable region of a heavy chain or a light chain of a target monoclonal antibody, or a portion thereof, and/or an Fab region or a portion thereof, in some cases, a portion of a constant region (for example, an Fc region), and preferably, a region including at least a CDR (CDR1, CDR2, and/or CDR3) region or a portion thereof. By subjecting the peptide fragment characteristic and specific to the antibody obtained thereby to mass spectrometry, accurate identification and quantification of the target antibody can be performed. The method of the present invention is applicable regardless of a kind of an antibody. Therefore, without being limited to the above exemplified antibodies, the method of the present invention is also applicable to newly developed monoclonal antibodies and the like.

When the CDR regions of an antibody are determined, the three CDR regions can be determined by confirming an amino acid substitution frequency distribution of a target antibody, multiple alignments of amino acid sequences between a target antibody and other antibodies, cross reactivity between a target antibody and an endogenous antibody, and the like. The term "CDR (region)" in the present specification refers to the CDR1 (region), the CDR2 (region), the CDR3 (region), or a combination of two or more of the CDR regions.

When an antibody drug (usually a monoclonal antibody) is administered to a patient at a clinical site, it is required to accurately measure an amount of the antibody in blood, and thereby a treatment plan can be effectively used. Blood contains a large amount of immunoglobulin in addition to a target antibody. Therefore, serum or plasma is collected from blood containing the target antibody by conventional centrifugation or the like, and the obtained serum or plasma is used as a sample for determining a concentration of the target antibody in the blood. Further, when necessary, a tissue containing a target antibody or a cell to which a target antibody is bound via a membrane receptor is separated, and, by using a conventional technique such as crushing, centrifugation, affinity chromatography (for example, Protein G binding or Protein A binding), an antibody fraction can be separated and used as a sample.

An embodiment of the present invention may further include a process of separating a target antibody or a antibody population containing the target antibody from a biological sample (for example, blood).

In the present invention, by using LCMS, even for a biological sample such as blood, plasma, serum, a tissue, or a cell, a qualitative and quantitative measurement of a target monoclonal antibody is possible. In this case, it is necessary that CDR sequences of the monoclonal antibody have been determined prior to analysis.

### <Porous Body>

A porous body (for example, "antibody Fc immobilization resin") used in the method of the present invention is not particularly limited as long as the porous body has a large number of pores, and activated carbon, a porous membrane, porous resin beads, metal particles, and the like, can be used. Among these, those capable of site-specifically binding an antibody are particularly preferable.

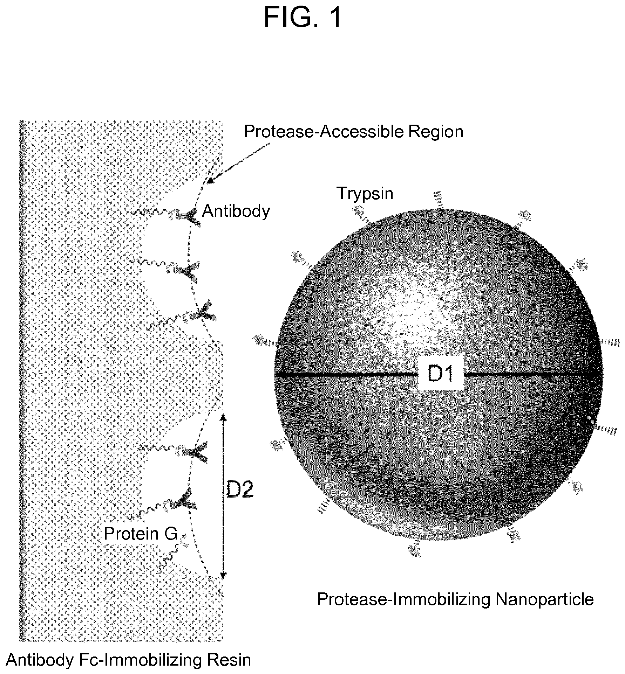

Fig. 1 illustrates hemispherical pores. However, the pores are not particularly limited in shape. Further, as in a case of a porous membrane, a porous body having pores formed penetrating the porous body can also be used. A size of a pore of a porous body is not particularly limited, and is preferably determined by considering a molecular diameter of an antibody and the like such that, when an antibody is immobilized, a part to be selectively digested is positioned near a surface layer of a pore. An average pore diameter (D2) of a porous body is appropriately set, for example, in a range of about 10 nm - 200 nm and in a range smaller than an average particle size (D1) of nanoparticles. The average pore diameter (D2) of a porous body is, for example, preferably about 20 nm - 200 nm, and more preferably about 30 nm - 150 nm. In order to immobilize an Fc region of an antibody in a pore and to regioselectively protease-digest an Fab region, a pore diameter of a porous body is preferably 30 nm - 150 nm, more preferably 40 nm - 120 nm, far more preferably 50 nm - 100 nm, and particularly preferably about 100 nm.

In the method of the present invention, a monoclonal antibody to be measured is immobilized in pores of a porous body. By immobilizing an antibody in pores and allowing the antibody to exist in a fine environment such as an interface between a solid phase and a liquid phase, the antibody is susceptible to denaturation, molecular fluctuation is subjected to perturbation, and probability of being attacked by a protease is increased. Further, in the present invention, as will be described later, by immobilizing a protease on nanoparticles, the protease is sterically stable and is in an environment in which self-digestion is unlikely to occur, and thus, stability of the protease is increased. Therefore, according to the method of the present invention, in addition to allowing regioselective protease digestion, high protease activity can be maintained.

In the present invention, a porous body in which a linker molecule that site-specifically interacts with an antibody is immobilized in a pore of the porous body is preferably used. Examples of the interaction between the antibody and the linker molecule include chemical bonding, hydrogen bonding, ionic bonding, complex formation, a hydrophobic interaction, a van der Waals interaction, an electrostatic interaction, a stereoselective interaction, and the like.

As a linker molecule, an antibody binding polypeptide, such as a Protein A, a Protein G or an IgG binding peptide that site-specifically binds with an antibody, is preferably used. The Protein A and the Protein G are known to site-specifically bind with an Fc region of an antibody (particularly IgG).

The Protein A is a protein of 46.7 kDa that exists on a cell wall of Staphylococcus aureus and specifically binds to an Fc region of immunoglobulin (particularly IgG). Regarding a binding property between the Protein A and human immunoglobulin, it is known that the Protein A strongly binds to human IgG1, IgG2, and IgG4, but hardly binds to human IgG3.

The Protein G is a protein that exists on a cell wall of group G streptococci (group G hemolytic streptococci), and it is known that the Protein G specifically binds to an Fc region of immunoglobulin (particularly, IgG) and also weakly binds to an Fab fragment.

The IgG binding peptide is a known polypeptide that specifically binds to IgG, as described, for example, in International Publication No. WO 2013/027796 A1, and is formed from about 13 - 17 amino acids.

By using a porous body in which these linker molecules are immobilized on a surface and in pores, an Fc region of an antibody is immobilized via the linker molecules in the pores, and a variable region or an Fab region of a heavy chain or a light chain of the antibody can be positioned near a surface layer of a pore. In this way, by controlling orientation of an antibody in a pore, regioselective digestion of a region by a protease becomes possible, the region including a variable region or an Fab region of a heavy chain or a light chain of the antibody, preferably including the CDR (CDR1, CDR2, and/or CDR3) in the variable region or a portion thereof. Here, for example, when referring to "a portion of the CDR1," the term "a portion thereof" means a portion that includes at least one, preferably multiple consecutive amino acid residues in an amino acid sequence of the CDR1 region.

A size of a linker molecule is selected such that a selective cleavage site of an antibody is positioned near a surface layer of a pore. A molecular size in a state in which a linker molecule and an antibody are bound to each other is preferably about 0.2 times - 1.5 times, more preferably about 0.6 times - 1.2 times, far more preferably about 0.7 times - 1.1 times, and particularly preferably about 0.8 times - 1 times larger than the pore diameter of a porous body. When a linker molecule is not fixed to a porous body and an antibody is directly bound in pores, it is preferable that a molecular diameter of the antibody and a pore diameter of the porous body satisfy the above relation.

A porous body that can be suitably used in the present invention is not particularly limited. For example, Protein G Ultralink resin (manufactured by Pierce Corporation), Toyopearl, TSKgel (manufactured by (TOSOH Inc.), and the like can be used. For example, in the case of the Protein G Ultralink resin, it is known that 95% of Protein G bound to resin are in pores.

### <Immobilization of Antibody in Porous Body>

A method for immobilizing an antibody in pores of a porous body is not particularly limited. An appropriate method can be adopted according to characteristics of the antibody, the porous body or a linker molecule and the like. For example, when an antibody is immobilized in a porous body in which an antibody binding polypeptide such as a Protein A, a Protein G, or an IgG binding peptide is immobilized in pores in advance, by mixing a suspension of the porous body with a solution containing the antibody, the antibody can be easily immobilized in pores.

When a biological sample is blood, plasma or serum, an antibody population containing the target antibody can be bound to the inner surfaces of the pores of the porous body. Even in such a case, precise separation is possible, for example, by liquid chromatography (LC). Therefore, by combining with mass spectrometry, a peptide fragment characteristic of a target antibody can be qualitatively and quantitatively determined using LCMS analysis.

A quantitative ratio of a porous body to an antibody can be appropriately set according to a purpose. For example, when a quantitative analysis of an antibody is performed, it is desirable that substantially the entire amount of the antibody in a sample be immobilized in the porous body. Therefore, it is preferable that a quantitative ratio be set such that an amount of the porous body becomes excessive with respect to an estimated content of the antibody in the sample.

### <Nanoparticles>

In the method of the present invention, nanoparticles are used to immobilize a protease on surfaces of the nanoparticles and to control access of the protease to an immobilized antibody in pores of a porous body. Therefore, the nanoparticles have a larger average particle size (D1) than the average pore diameter (D2) of the porous body so as not to enter deep into the pores of the porous body (Fig. 1).

The nanoparticles are not particularly limited in shape. However, from a point of view of homogenization of access of the protease to the pores of the porous body, spherical nanoparticles are preferred. Further, it is preferable that the nanoparticles have high dispersibility, small diameter variations, and a uniform particle size.

The average particle size (D1) of the nanoparticles is in a range of 50 nm - 500 nm, and is more preferably 1.2 or more times, far more preferably 1.5 or more times, and particularly preferably 1.8 or more times the average pore diameter (D2) of the porous body. When efficiency of a protease digestion reaction between solid phases is considered, although it is not limited to the following ranges, the average particle size (D1) of the nanoparticles is preferably 1.5 times - 4 times, far more preferably 1.8 times - 2.5 times, for example about 2 times, larger than the average pore diameter (D2) of the porous body. For example, when the average pore diameter of the porous body is about 30 - 150 nm, the average particle size (D1) of the nanoparticles is preferably 100 nm or more, and more preferably 150 nm or more. When the average pore diameter of the porous body is about 50 nm - 100 nm, the average particle size of the nanoparticles is preferably 120 nm or more, more preferably 150 nm or more, and particularly preferably 170 nm or more. From a point of view of improving digestion efficiency by the protease, an upper limit of the average particle size (D1) of the nanoparticles is preferably 500 nm or less, and more preferably 300 nm or less.

A material of the nanoparticles is not particularly limited as long as the above protease can be immobilized on surfaces of the nanoparticles. A metal, a resin, or the like can be appropriately used as the material of the nanoparticles. Further, a metal coated with a resin, a resin coated with a metal, or the like can also be used.

As a kind of the nanoparticles, magnetic nanoparticles that can be dispersed or suspended in an aqueous medium and can be easily recovered from the dispersion or suspension by magnetic separation or magnetic precipitation separation are preferable. Further, from a point of view that aggregation is less likely to occur, magnetic nanoparticles coated with an organic polymer are more preferable. Examples of base materials of magnetic nanoparticles include ferromagnetic alloys such as iron oxide (magnetite (Fe₃O₄), maghemite (γ-Fe₂O₃)), and ferrite (Fe/M)₃O₄. In the ferrite (Fe/M)₃O₄, M means a metal ion that can be used together with an iron ion to form a magnetic metal oxide, and typically, Co²⁺, Ni²⁺, Mn²⁺, Mg²⁺, Cu²⁺, Ni²⁺ and the like are used. Further, examples of the organic polymer coating the magnetic nanoparticles include polyglycidyl methacrylate (poly GMA), a copolymer of GMA and styrene, polymethyl methacrylate (PMMA), polymethyl acrylate) (PMA), and the like. Specific examples of magnetic nanobeads coated with an organic polymer include FG beads, SG beads, Adembeads, nanomag, and the like. As a commercially available product, for example, FG beads (polymer magnetic nanoparticles having a particle size of about 200 nm obtained by coating ferrite particles with polyglycidyl methacrylate (poly GMA)) manufactured by Tamagawa Seiki Co., Ltd. (Tokyo, Japan) is suitably used.

In order to suppress adsorption of a nonspecific protein and to selectively immobilize a protease, it is preferable that the nanoparticles be modified with spacer molecules capable of binding to the protease. By immobilizing a protease via a spacer molecule, desorption of the protease from surfaces of the nanoparticles is suppressed, and regioselectivity of protease digestion is improved. Further, by adjusting a molecular size of a spacer, a protease can be caused to selectively access a desired position of an antibody, and regioselectivity can be improved.

A spacer preferably can bind to protease and does not inactivate a protease. From a point of view of controlling an access range of a protease immobilized on surfaces of nanoparticles, a spacer preferably has a small molecular diameter. The molecular diameter of the spacer is preferably 5 nm or less, more preferably 3 nm or less, and far more preferably 2 nm or less. Further, a molecular weight of the spacer is preferably 2000 or less, more preferably 1500 or less, and far more preferably 1000 or less.

A spacer molecule having the above molecular diameter and capable of immobilizing a protease is preferably a non-protein, and is preferably a molecule having a functional group at a terminal, examples of the functional group including an amino group, a carboxyl group, an ester group, an epoxy group, a tosyl group, a hydroxyl group, a thiol group, an aldehyde group, a maleimide group, a succinimide group, an azide group, a biotin, an avidin, and a chelate. For example, for immobilization of trypsin, a spacer molecule having an activated ester group is preferred. Further, of a spacer molecule, as a spacer arm portion other the functional group, a hydrophilic molecule can be used, examples of the hydrophilic molecule including polyethylene glycol and its derivatives, polypropylene glycol and its derivatives, polyacrylamide and its derivatives, polyethyleneimine and its derivatives, poly (ethylene oxide) and its derivatives, poly (ethylene terephthalic acid) and its derivatives, and the like.

Nanoparticles surface-modified with such spacer molecules are also commercially available, and these commercially available nanoparticles can be used. For example, nanoparticles modified with a spacer molecule having an ester group (active ester group) activated with N-hydroxysuccinimide is commercially available under a trade name of "FG beads NHS" (Tamagawa Seiki Co., Ltd.). The FG beads NHS has a particle size of about 200 nm ± 20 nm, and is very homogeneous as nanoparticles.

### <Protease>

In the method of the present invention, a protease cleaves an antibody immobilized in pores of a porous body at a specific amino acid sequence site to generate a peptide fragment that comprises a CDR region or a portion thereof in a variable region of a heavy chain or a light chain of the antibody.

In the present invention, a kind of a protease to be immobilized on nanoparticles may be appropriately selected according to a kind of a protein to be quantified or identified by mass spectrometry, and is not limited. Examples of the protease include trypsin, chymotrypsin, lysyl endopeptidase (Lys-C), V8 protease, Asp N protease (Asp-N), Arg C protease (Arg-C), papain, pepsin, dipeptidyl peptidase, and the like. Two or more kinds of these proteases can be used in combination.

Among the above proteases, trypsin is particularly preferably used in the present invention. Trypsin has high substrate specificity and has Lys or Arg at a C terminal of a peptide after cleavage and thus can homogenize a charge amount and charge localization of a peptide, and is particularly suitable for preparation of samples for mass spectrometry. Further, trypsin has a small molecular diameter (about 3.8 nm) and an active site exists inside a molecule. Therefore, a region where the active site can access an antibody is restricted, and regioselectivity of protease digestion can be improved.

When a peptide fragment of an antibody after protease digestion is subjected to mass spectrometry as a measurement sample, it is preferable to use a protease having less self-digestion and high cleavage sequence selectivity. When a commercially available protease is used, it is preferable to use a protease of a mass spectrometry grade or a sequencing (sequence) grade. For example, a native trypsin derived from a living body has remaining self-digestion activity or has a pseudo trypsin showing chymotrypsin-like activity, and thus is known to have low cleavage site specificity and activity. Therefore, trypsin of a mass spectrometry grade having improved resistance to self-digestion by subjecting lysine residue of the trypsin to reductive methylation is commercially available.

Examples of the protease that can be suitably used in the method of the present invention include Trypsin Gold (manufactured by Promega Corporation), Trypsin TPCK-Treated (manufactured by Sigma Corporation), and the like.

### <Immobilization of Trypsin on Nanoparticles>

A method for immobilizing a protease on surfaces of nanoparticles is not particularly limited. An appropriate method can be adopted according to characteristics of the protease and the nanoparticles (or spacer molecules modifying the surfaces of the nanoparticles). For example, when the protease is immobilized on spacer-modified surfaces of nanoparticle, by mixing a suspension of the nanoparticles and a solution containing the protease, the protease can be immobilized on the surfaces of the nanoparticles. An amine coupling method of the nanoparticles and the protease via the functional groups of the spacer molecules is preferable. For example, a carboxyl group modifying surfaces of nanoparticles can be esterified with N-hydroxysuccinimide (NHS) to form an activated ester group to which an amino group of a protease can be bound. This coupling reaction can be performed in the presence of carbodiimide as a condensing agent, examples of the carbodiimide including 1-ethyl-3-(3-dimethylaminopropyl) carbodiimide (EDAC), N,N'-dicyclohexylcarbodiimide (DCC), bis(2,6-diisopropylphenyl) carbodiimide (DIPC), and the like. Further, an amino group of a protease may be bound to an amino group modifying surfaces of nanoparticles using a cross-linking agent such as glutaraldehyde, bifunctional succinimide, bis(sulfosuccinimidyl) suberate (BS3), sulfonyl chloride, maleimide, and pyridyl disulfide.

The coupling method of the nanoparticles and the protease via the functional groups of the spacer molecules can be performed by a simple operation of adding a protease solution to a suspension of the nanoparticles and mixing and stirring the mixture under certain conditions. The reaction conditions are not particularly limited. However, for example, the suspension of the nanoparticles, to which the protease is added, is stirred at a temperature of 1 - 10 °C at pH 7.0 at 50 - 200 rpm for 0.5 - 1 hour.

After the protease is immobilized on the surfaces of the nanoparticles, an active portion that is not bound to the protease on the surfaces of the nanoparticles can be inactivated. For example, when spacer molecules on which the protease is not immobilized exist on the surfaces of the nanoparticles, problems may occur such as that the unbound spacer molecules bind to contaminants in the sample and adversely affect protease digestion, and that peptide fragments produced by protease digestion are immobilized on the nanoparticles. After the protease is immobilized, by blocking unbound spacer molecules, such problems are suppressed. As a method for inactivating the active portion unbound to the protease, chemical modification is preferred. For example, an activated ester group can be inactivated by reacting with a primary amine to form an amide bond.

### <Protease Digestion>

According to the present invention, by bringing the porous body, in which the target monoclonal antibody is immobilized, and the nanoparticles, on surfaces of which the protease is immobilized, into contact with each other in a liquid by stirring or tapping rotation stirring at a low speed of less than 100 rpm so as to maintain a uniform dispersion, the antibody is protease-digested, and a peptide fragment that comprises an Fab region or a variable region of the antibody or a portion of the region is produced with an increased yield from the antibody. The conditions including the stirring in the present method can increase the yield of the target peptide fragment by about 2 - 5 times as compared to a case of vortex stirring (rotation speed: 100 rpm).

The term "liquid" used in the present specification means a medium for causing a substrate (solid phase) and an enzyme (solid phase) to contact and react with each other in a liquid phase, and means an aqueous medium suitable for a protease digestion reaction.

A necessary condition for the protease digestion in the present invention is that the protease digestion reaction is performed by bringing the porous body in which the target monoclonal antibody is immobilized and the nanoparticles on which the protease is immobilized into contact with each other in a liquid by stirring or tapping rotation stirring at a low speed of less than 100 rpm, more preferably 50 rpm or less, and far more preferably 20 rpm or less so as to maintain a uniform dispersion. When the speed is in such a range, an effect that is the same as or close to that of examples to be described later can be obtained.

The "stirring" in the present specification can be performed in any stirring mode as long as the above condition is satisfied. Examples of the stirring include, but not limited to, stirring using a rotary mixer, stirring using a stirrer, stirring using a vortex mixer, and the like, which can be adjusted to a low speed.

The term "tapping rotation stirring" used in the present specification means stirring by gentle rotation at a speed within the above range accompanied by periodic tapping. In such stirring, the term "gentle rotation" refers to, for example, rotation at a speed of about 3 - 10 rpm, and the term "tapping" refers to a momentary action such as flipping or imparting a shock or an impact (frequency: for example, 1 - 5 times, preferably 2 - 4 times, per minute). As a result, while both the antibody-immobilizing porous body and the protease-immobilizing nanoparticles retain a uniformly dispersed state, the protease on the nanoparticles substantially preferentially attacks an Fab region or a variable region of a heavy chain or a light chain on an N terminal side of the monoclonal antibody in the pores of the porous body, and the protease digestion reaction efficiency is increased.

Specifically, the tapping rotation stirring can be performed using a stirring device such as a commercially available tapping rotary mixer, for example, a shock-attached (tapping) rotary mixer NRC-20D (Nissin Rika Corporation (Tokyo, Japan); rotation speed: 1 - 15 rpm; tapping 2 times/minute).

A further necessary condition is that the liquid used in the reaction is a salt-free buffer solution. The salt means any salt other than a buffer component, in particular sodium chloride (NaCl) and potassium chloride (KCl). When a salt is present, a repulsive force between particles (solid phase) weakens and collisions between the particles tend to occur, and thus, protease digestion becomes difficult to proceed.

As another condition for the protease digestion, generally, depending on a kind of a protease, in a buffer solution adjusted to an optimal pH or a pH near the optimal pH, an optimal temperature or a temperature near the optimal temperature is selected. When the protease is trypsin, the buffer solution is usually a Tris buffer or the like at pH 7.5 - 9.0, for example, at pH 8.0, and the temperature is usually about 35 - 40 °C, for example, about 37 °C. In the method of the present invention, a reaction incubation time can be, but not limited to, about 2 - 20 hours. However, since shortening a total analysis time is also an important factor, by optimizing a ratio of an amount of the antibody to an amount of the protease or a ratio of the pore diameter of the porous body to the particle size of the nanoparticles, the reaction time can be shortened to, for example, 1 - 2 hours or even less. In particular, when the ratio of the pore diameter of the porous body to the particle size of the nanoparticles is determined, by setting the ratio such that the protease can selectively attack the CDR (CDR1, CDR2, and/or, CDR3) regions of the variable regions of the heavy chains and the light chains of the antibody bound in the pores of the porous body, the reaction time can be shortened.

A quantitative mixing ratio of the porous body (on which the antibody is immobilized) to the nanoparticles (on the surfaces of which the protease is immobilized) is not particularly limited. or example, substrate:protease is about 100:1 - 20:1 (weight ratio). In the present invention, by a combination of the porous body and the nanoparticles, access between the antibody and the protease is physically restricted. Therefore, as compared to general protease digestion, it is preferable to increase the amount of the protease. For example, monoclonal antibody:protease is preferably about 30:1 - 3:1, more preferably about 15:1 - 4:1, and far more preferably about 10:1 - 5:1.

In the method of the present invention, digestion by an immobilized protease is performed in a state in which the monoclonal antibody is immobilized on the porous body. Since the peptide fragments produced by the protease digestion exist in the liquid phase, target peptide fragments can be regioselectively obtained without performing an antibody elution or denaturation operation. According to the method of the present invention, as compared to a conventional method, it is possible to achieve an increase in a yield of a peptide fragment comprising a CDR (i.e., CDR1, CDR2, and/or, CDR3) region, or a portion thereof, of a variable region of a heavy chain or a light chain of an antibody, preferably, regioselectively cleaved with a simple operation.

More specifically, for example, a C-terminal side of an antibody is noncovalently bound to a Protein G on a Protein G-immobilizing resin having a pore diameter of about 90 - 120 nm, and a variable region of the antibody is always oriented to a solution side. Next, a protease is immobilized on surfaces of nanoparticles having a particle size of about 180 - 240 nm. By limiting contact of a protease with the antibody, it is possible to form a reaction field in which a protease decomposition reaction of an antibody is selectively performed in a variable region. Further, by using surfaces of nanoparticles having very large relative surface areas as a protease reaction field, probability of contact with an antigen can be increased.

In this regard, in Fig. 1, since the average particle size (D1) of the nanoparticles is larger than the average pore diameter (D2) of the porous body, the nanoparticles can access near surface layers of the pores but cannot access deep into the pores. Along with this, the protease immobilized on the surfaces of the nanoparticles also cannot access deep into the pores. In Fig. 1, dotted lines near the pores each represent a boundary of a protease accessible region. In this way, protease access to an antibody in the pores is regiospecifically restricted and a relative access probability to a liquid phase side of the antibody (particularly the variable region or the Fab region) is increased. As a result, an antibody can be regioselectively protease-digested to obtain a peptide fragment characteristic to the antibody.

In the examples to be described later, protease digestion reactions according to the method of the present invention were carried out using trastuzumab and bevacizumab, which are antibody drugs, as specific examples. Results of the examples showed a trend that a yield of a peptide fragment in the case of tapping rotation stirring (rotation speed: 3 - 10 rpm; tapping number: 2 times/minute) was increased by about 3 - 5 times in the case of trastuzumab and by about 1.4 - 4 times in the case of bevacizumab, as compared to the case of vortex stirring (rotation speed: 100 rpm), when a reaction scale was about 200 µL, an amount of trypsin was 3 µg, an antibody concentration was 1.1 µg/mL - 10 µg/mL (100 µL used), temperature was 37 °C, and a reaction time was 6 hours. Further, it was confirmed that, by protease digestion of trastuzumab and bevacizumab according to the present method, peptide fragments comprising CDR regions, or portions thereof, characterizing these antibodies were collected with increased yields. Examples of such peptide fragments are as follows.

In the case of digestion of trastuzumab by trypsin, the examples of the peptide fragments include TNGYTR (SEQ ID No: 1; a portion of VH CDR2), FTISADTSK (SEQ ID No: 2; a portion of VH CDR3), ASQDVNTAVAWYQQKPGK (SEQ ID No: 3; a portion of VL CDR1), NTAYLQMNSLR (SEQ ID No: 4;a portion of VH CDR3), GLEWVAR (SEQ ID No: 5; a portion of VH CDR2), and DTYIHWVR (SEQ ID No: 6; a portion of VH CDR1).

In the case of digestion of bevacizumab by trypsin, the examples of the peptide fragments include STAYLQMNSLR (SEQ ID No: 7; a portion of VH CDR3), FTFSLDTSK (SEQ ID No: 8; a portion of VH CDR3), GLEWVGWINTYTGEPTYAADFKR (SEQ ID No: 9; a portion of VH CDR2, and CDR2), VLIYFTSSLHSGVPSR (SEQ ID No: 10; a portion of VL CDR2, CDR2, and a portion of CDR3).

In order to subject a target peptide fragment obtained by the protease digestion to mass spectrometry, it is necessary to remove the porous body and the nanoparticles from a reaction system. This can be achieved by subjecting a sample after the protease digestion to filtration, centrifugation, magnetic separation, dialysis, and the like. For example, by filtration using a filtration membrane made of polyvinylidene fluoride (PVDF) (low-binding hydrophilic PVDF having a hole diameter of 0.2 µm manufactured by Millipore Corporation), the porous body and the nanoparticles can be easily removed.

### 2. Quantification of Antibody Peptide Fragment

By analyzing a sample containing the above-obtained peptide fragment using chromatography or mass spectrometry, identification and quantification of the antibody can be performed. In the present invention, since a substrate protein is regioselectively subjected to a protease treatment, the number of kinds of peptide fragments contained in a sample is reduced. Therefore, conditions of analysis by mass spectrometry or the like can be easily set. In performing analysis, a sample may be used for the analysis after being subjected to treatments such as desalting, solubilization, extraction, concentration, and drying when necessary.

Mass spectrometry is suitable for identification and quantification of a substrate protein from a peptide fragment produced by protease digestion. Mass spectrometry can determine an amino acid sequence and thus can determine whether or not a peptide fragment is a peptide fragment derived from a specific protein such as an antibody. Further, based on a peak intensity, a concentration of a peptide fragment in a sample can be determined.

An ionization method in mass spectrometry is not particularly limited, and an electron ionization (EI) method, a chemical ionization (CI) method, a field desorption (FD) method, a fast atom collision (FAB) method, a matrix assisted laser desorption ionization (MALDI) method, an electrospray ionization (ESI) method, and the like can be adopted. A method for analyzing an ionized sample is also not particularly limited, and a method of a magnetic field deflection type, a quadrupole (Q) type, an ion trap (IT) type, a time of flight (TOF) type, a Fourier transform ion cyclotron resonance (FT-ICR) type, or the like can be appropriately determined according to the ionization method. Further, MS/MS analysis or multistage mass spectrometry of MS3 or higher can also be performed using a triple quadrupole mass spectrometer or the like.

For purposes such as more reliably separating the peptide fragment and improving analysis accuracy, a sample before being subjected to mass spectrometry is subjected to separation and concentration using liquid chromatography (LC), solid phase extraction (SPE) or the like. When separation of a sample is performed using LC, LC/MS having LC may be used as a first stage of mass spectrometry, and an eluate from LC may be directly ionized and subjected to mass spectrometry. Analysis can also be performed using LC/MS/MS or LC/MSn combining LC and tandem mass spectrometry. Further, the eluate from the LC may be collected once and then subjected to mass spectrometry. An LC column or an SPE carrier is not particularly limited, and a hydrophobic column such as C30, C18, C8, and C4 generally used in peptide analysis, a carrier for hydrophilic affinity chromatography, and the like can be appropriately selected and used. LC is useful for distinguishing and separating a peptide fragment characteristic of a target antibody from peptide fragments derived from other antibodies that are derived from a blood sample and are bound to a porous body.

Based on a mass spectrometry result, in order to identify a protein such as an antibody, an existing database can also be used. In the present invention, a peptide fragment obtained by regiospecifically protease-digesting a substrate protein such as an antibody is used. Therefore, a database search hit ratio and data accuracy are increased. Further, it is also possible to identify an antibody, which is a substrate protein, by identifying an amino acid sequence of a peptide fragment using multistage mass spectrometry or the like. For example, when an amino acid sequence of a peptide fragment comprising at least a portion of an amino acid sequence of a complementarity-determining region (CDR) having an amino acid sequence specific to an antibody is determined, the antibody can be identified.

When detection and quantification of an antibody is performed based on a detection result of a specific peptide fragment comprising a sequence of a CDR or a portion thereof, the number of amino acid residues of the peptide to be detected is preferably about 5 - 30, and more preferably about 7 - 25. When the number of the amino acid residues is excessively small, it is difficult to distinguish between contaminants and peptide fragments derived from other sites of the same protein, and this can cause erroneous detection and the like. Further, when the number of the amino acid residues is excessively large, for reasons such as that ionization becomes difficult, detection may become difficult and quantitativeness may decrease.

When a concentration of an antibody, which is a substrate protein, is quantified, an amount of the antibody can be calculated based on a peak area or a peak intensity of a detected peptide fragment ion (in the case of multistage MS, a cleaved ion obtained by cleavage of a peptide fragment ion). Further, it is also possible to use an internal standard labeling stable isotopes when performing quantification. For example, based on a correlation between a predetermined calibration curve and a peak area, or a correlation between a peak area derived from an internal standard added to a sample and a peak area derived from the sample, a concentration of peptide fragments in the sample is calculated, and, based on the concentration of the peptide fragments, an amount and a concentration of the antibody are calculated.

An example of conditions of LCMS is shown below. However, it is desirable to set conditions suitable for a device to be used.
(HPLC Conditions (Nexera LC30A Liquid Chromatography System (Shimadzu Corporation, Kyoto, Japan)))
Solvent A: 0.1% formic acid; solvent B: 0.1% formic acid + acetonitrile
Flow rate: 0.5 ml/minute
Gradient time: 1% B (1.5 minutes), 1 - 40% B gradient (5 minutes), 95% B (5 minutes), 1% B (5 minutes)
Column: L-column 2 ODS, 2 x 50 mm (Chemical Substance Evaluation and Research Organization, Tokyo, Japan))
Column temperature: 50 °C
(MS Interface Conditions (LCMS-8050 (Shimadzu Corporation)))
Nebulizer gas: 3 L/minute
Heating gas: 10 L/minute
Drying gas: 10 L/minute
Interface temperature: 300 °C
DL temperature: 250 °C
Heat block temperature: 400 °C

### 3. Peptide Fragment Preparation Kit

The present invention further provides, according to a third aspect, a kit for use in the method described above in Section 1. The kit includes: (i) a porous body having pores capable of immobilizing a target monoclonal antibody, (ii) nanoparticles immobilizing a protease on surfaces thereof, and (iii) an instruction manual for instructing a user to bring the target monoclonal antibody and the protease into contact with each other in a liquid by stirring or tapping rotation stirring at a low speed of 100 rpm or less so as to maintain a uniform dispersion, an average particle size of the nanoparticles being larger than a pore diameter of the porous body.

In one embodiment, the kit can further include an affinity means for separating and purifying an antibody from a biological sample or the like.

Such an affinity means is, for example, an affinity column obtained by covalently binding Protein A or Protein G to an agarose gel, such as one obtained by binding an antibody binding polypeptide to solid phase gel particles, and, usually, may be a commercially available product.

In another embodiment, the porous body can have a linker molecule bound to surfaces of particles and inner surfaces of pores. A preferable linker molecule is an antibody binding polypeptide as described above, and, specifically, is Protein A, Protein G, IgG binding peptide or the like.

The porous body and the nanoparticles included in the kit of present invention are as described above. Further, the instruction manual describes procedures and conditions for enabling an increase in a yield of a peptide fragment derived from a target antibody according to the method of the present invention.

According to the usage manual, a user can obtain a target peptide fragment with an increased yield based on regiospecific protease digestion of the target antibody.

In another embodiment, a preferred protease is trypsin. However, various proteases described and exemplified above can be immobilized.

Trypsin has high substrate specificity and has Lys or Arg at a C terminal of a peptide after cleavage and thus can homogenize a charge amount and charge localization of a peptide, and has advantages such as being particularly suitable for preparation of a sample for mass spectrometry, and being able to cut out a peptide fragment of a moderate size since a basic amino acid is moderately present in an amino acid sequence of the variable regions of the heavy chains and light chains of an antibody.

### EXAMPLES

In the following, experimental examples are described in which trastuzumab (trade name: Herceptin) and bevacizumab (trade name: Avastin) were selected as specific examples of monoclonal antibodies, and peptide fragments, which were digestion products obtained by protease-digesting the antibodies under conditions of using tapping rotation stirring (the present invention) or vortex stirring (comparison), were subjected to mass spectrometry to determine yields. The technical scope of the present invention is not limited to the following specific examples.

### <Reagents and the like>

In the following, unless otherwise specified, "%" represents "% by weight." Reagents used in the present experimental examples are as follows.
Trypsin (sequence grade, promega)
Lysyl endopeptidase (mass spectrometry grade, Wako Pure Chemical Industries (Osaka, Japan))
2-morpholinoethanesulfonic acid, (MES, Doujin Chemistry (Kumamoto, Japan))
2-[4-(2-hydroxyethyl)-1-piperazinyl] ethanesulfonic acid (HEPES, Doujin Chemistry)
Tris (hydroxymethyl) aminomethane (Tris, Wako Pure Chemical Industries)
Organic solvents and the like that are other than the above and are not specifically described were obtained from Wako Pure Chemical Industries.

Further, as buffers, the following buffer solutions, for each of which pH was adjusted using a precision pH meter, were used.
MES buffer: 25 mM MES-NaOH, pH 5.5
HEPES buffer: 25 mM HEPES-NaOH, pH 7.0
Ethanolamine buffer: 1 M ethanolamine-HCl, pH 8.0
Tris buffer: 25 mM Tris-HCl, pH 8.0

### <Preparation of Antibody Immobilizing Porous Body>

5 µL of a resin beads suspension (Pierce Biotechnology, Protein G UltraLink resin, average particle size: 100 µm, pore diameter: 50-100 nm), in which Protein G was bound to surfaces of porous beads, was added to 200 µL of an MES buffer, and an antibody solution was added thereto. Thereafter, the mixture was slowly stirred at room temperature for about 1 hour, and the antibody was bound to the Protein G on the surfaces of the resin beads and was immobilized. Thereafter, centrifugation (15000 rpm, 1 minute) at 4 °C was performed to precipitate the resin beads, and a supernatant was removed. Thereafter, washing with a Tris buffer and centrifugation were repeated twice, and the porous beads were suspended in a Tris buffer (200 µL). As the antibody solution, solutions of trastuzumab (trade name: Herceptin, 20 mg/mL, Chugai Pharmaceutical) and bevacizumab (trade name: Avastin, 20 mg/mL, Chugai Pharmaceutical) were used.

### <Preparation of Protease-Immobilizing Nanoparticles>

Protease-immobilizing nanoparticles (FG beads NHS manufactured by Tamagawa Seiki (Tokyo, Japan)), obtained by modifying surfaces of nanoparticles having an average particle size of 190 nm with a spacer (see the following chemical formula (where L is a binding site to a surface of a nanoparticle); spacer length: 1 nm) of which a carboxy group is activated with N-hydroxysuccinimide, were used.

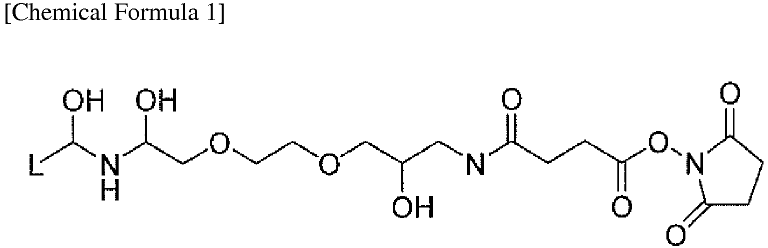

50 µl of an isopropanol suspension of FG beads was centrifuged at 4 °C (15000 rpm, 5 minutes) to precipitate the nanoparticles, and a supernatant was removed, and thereafter, washing with methanol was performed. A solution obtained by dissolving a solution containing 50 µg of a protease in 200 µL of a HEPES buffer was added to the nanoparticles to suspend the nanoparticles. In forming the suspension, an ultrasonic treatment was performed for a few seconds so that a temperature of the suspension did not rise.

The suspension of the nanoparticles was stirred at 4 °C for 30 minutes, and was centrifuged at 4 °C (15000 rpm, 5 minutes) to precipitate the nanoparticles, and a supernatant was removed. Thereafter, 200 µL of an ethanolamine buffer was added to suspend the beads. The mixture was stirred at 4 °C for 30 minutes, and excess N-hydroxy guanosuccinimide groups on the surfaces of the nanoparticles were blocked with ethanolamine. Thereafter, centrifugation (15000 rpm, 5 minutes) at 4 °C was performed to precipitate the nanoparticles, and a supernatant was removed. Thereafter, washing with a Tris buffer and centrifugation were repeated twice, and the nanoparticles were suspended in a Tris buffer (100 µL). A protease concentration in this suspension is 0.5 µg/µL.

### [Experiment 1: Confirmation of Antibody Immobilization Amount in Porous Body]

In preparing an antibody-immobilizing porous body, an amount of a Protein G-binding resin beads suspension was varied in a range of 0 - 20 µL with respect to 100 µg of IgG, a supernatant was analyzed using SDS-PAGE electrophoresis, and an approximate amount (remaining antibody amount) of unbound IgG remaining in the supernatant was determined from the number of electrophoretic image band pixels. It was confirmed that, along with an increase in the amount of the Protein G-binding resin beads, there was a tendency that the remaining antibody amount was reduced, the remaining antibody amount was about 3% when the amount of the Protein G-binding resin beads was 10 µL, and a catalog spec of the Protein G-binding resin beads was substantially reproduced.

### [Experiment 2: Examination of Quantitative Ratio of Antibody to Protease]

An IgG-immobilizing porous body suspension (Protein G-IgG) and the protease-immobilizing nanoparticles (FG beads-Tripsin) were mixed, and protease digestion was performed while the mixture was gently stirred at 37 °C for 15 hours. Thereafter, the resin was precipitated by centrifugation (15000 rpm, 5 minutes) at 4 °C, and a liquid phase (supernatant) was collected. The above experiments were performed by varying the amount of protease-immobilizing nanoparticles such that the amount of the protease was 5 µg (level 1), 10 µg (level 2), or 25 µg (level 3). At levels 4 - 6, similar experiments were performed using a porous body (Protein G UltraLink resin (Thermo Fisher Scientific)), in which IgG was not immobilized, in place of the IgG-immobilizing porous body suspension. Further, at levels 7 and 8, only the protease-immobilizing nanoparticles (FG beads-Tripsin) were incubated at 37 °C for 15 hours without using a porous body.

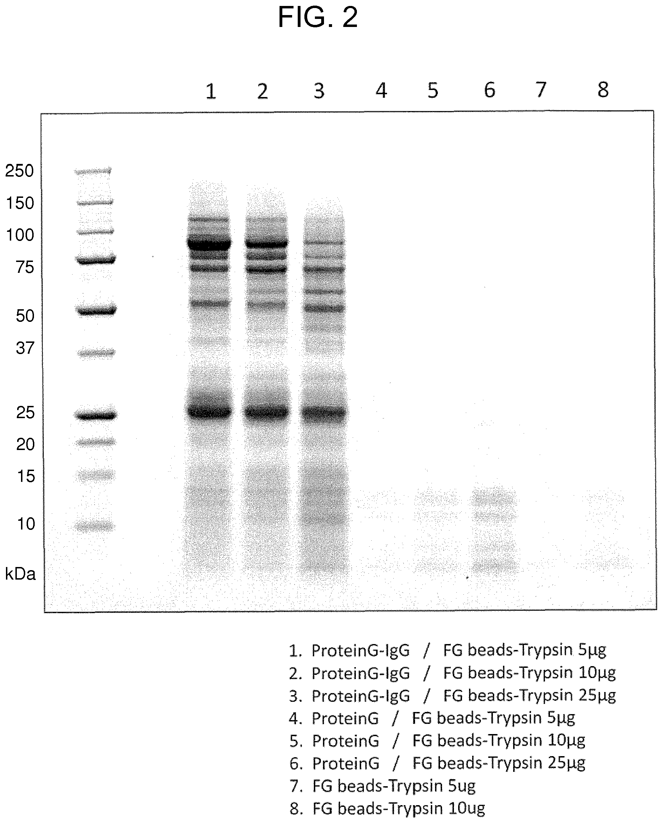

Table 1 shows the levels of the experiments. A weight (µg) in Table 1 is an amount of a protein (IgG or trypsin) in a sample. Fig. 2 illustrates an SDS-PAGE electrophoresis image of an obtained supernatant. In Fig. 2, a leftmost lane is a molecular weight marker.

**[Table 1]**

| | Protein G - IgG (µg) | FG beads - Trypsin (µg) |
|---|---|---|
| 1 | 100 | 5 |
| 2 | 100 | 10 |
| 3 | 100 | 25 |
| 4 | Protein G only | 5 |
| 5 | Protein G only | 10 |
| 6 | Protein G only | 25 |
| 7 | - | 5 |
| 8 | - | 10 |

Further, protease digestion products were analyzed by mass spectrometry.

Supernatants of the levels 1 - 6 were analyzed using MALDI-TOFMS (Shimadzu Corporation, AXIMA Resonance MALDI-QIT TOF MS). First, trifluoroacetic acid was added to 20 µL of a supernatant such that a final concentration became 0.5%. Purification was performed using a hydrophobic resin-filled chip (Millipore, ZipTip uC18). Thereafter, elution with 1 µL was performed twice. The eluate was directly applied onto a MALDI stainless steel target and was air-dried in a clean bench. After the air-drying, 1 µL of 10 mg/mL 2,5-dihydroxybenzoic acid solution (DHBA, Shimadzu GLC, water/acetonitrile = 50/50) was overlaid. An m/z of a device was performed using Angiotensin II peptide (m/z = 1046.54, Sigma-Aldrich) and ACTH fragment peptide (m/z = 2465.20, Sigma-Aldrich).

All of bands of the levels 4 - 6 in Fig. 2 were the same as bands of the levels 7 and 8. Further, peaks at m/z = 842, 1045, 2211 and 2283 detected at the levels 4 - 6 are all self-digestion fragments of trypsin. From these results, it is clear that the Protein G is not digested even when the protease-immobilizing nanoparticles and the Protein G porous body are brought into contact with each other. This is believed to be due to that the trypsin immobilized on the surfaces of the nanoparticles cannot access the Protein G in the pores because the pore diameter of the porous body is smaller than the particle size of the protease-immobilizing nanoparticles.

At the levels 1 - 3, peaks at m/z = 835, 913, 1187, 1287, 1510, 1678 and 1946 are detected in addition to the self-digestion fragments of the trypsin, and it was confirmed that these are all trypsin digestion peptide fragments of IgG. From this result, it was shown that IgG can be selectively digested without digesting the Protein G in the porous body by bringing the porous body, on which IgG is immobilized, and the nanoparticles, on which trypsin is immobilized, into contact with each other.

Comparing the levels 1 - 3 in Fig. 2, it is clear that, as the amount of the trypsin increases, bands on a polymer side decreases, and the digestion reaction of the antibody is well advanced. On the other hand, as the amount of the trypsin increases, self-digestion also becomes prominent. Based on these results, the level 2 (substrate:protease ratio = 10:1) was set as a standard condition, and the following conditions were examined.

A general protease digestion condition is that (substrate protein):(protease) = 100:1 - 20:1. In the present method, by a combination of the porous body and the nanoparticles, access between the substrate protein and the protease is physically restricted, and thus, as compared to general protease digestion, the amount of the protease increases, and an optimal substrate:protease ratio is estimated to be about 10:1 - 5:1.

### [Experiment 3: Evaluation of Collected Peptide against Digestion Time]

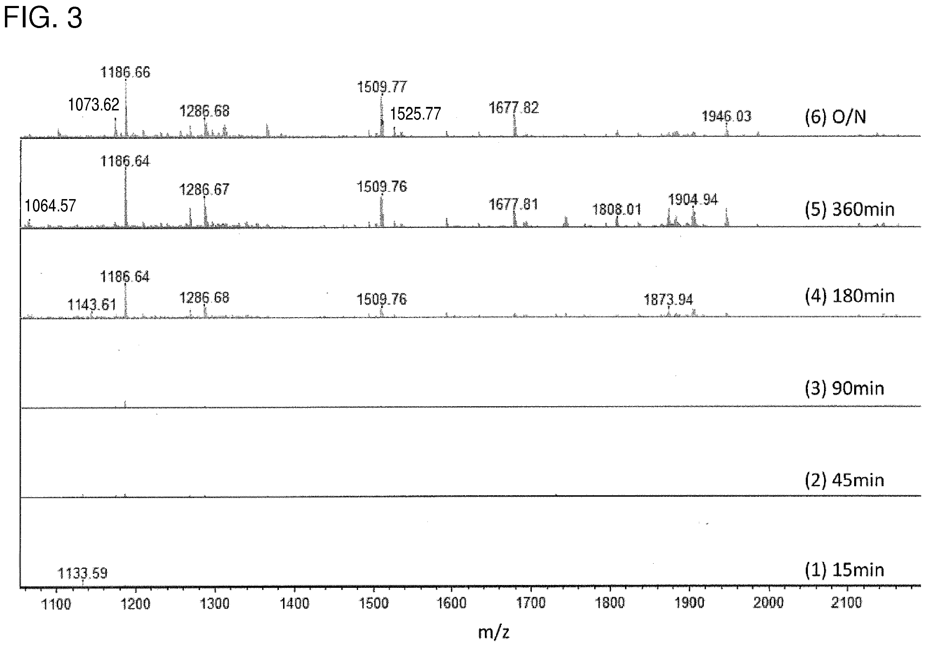

The condition of the level 2 of the experiment 2, that is, the IgG-immobilizing porous body suspension (solid phase amount: 100 µg) and the protease-immobilizing nanoparticles (trypsin solid phase amount: 10 µg) were mixed, and digestion time at 37 °C was set to (1) 15 minutes, (2) 45 minutes, (3) 90 minutes, (4) 180 minutes, (5) 360 minutes, and (6) 15 hours (overnight, O/N). Other than that, the trypsin digestion was performed in the same manner as in the experiment 2, and obtained samples were subjected to mass spectrometry. Fig. 3 shows MS spectra.

As shown in Fig. 3, it is clear that, as the digestion time increases, peaks of the peptide fragments are increased, and collection amounts of the peptide fragments are increased. When (5) 360 minutes and (6) overnight are compared to each other, the case of overnight has a higher collection rate, and, in particular, there is a tendency that accumulation of fragments susceptible to protease digestion such as those of m/z = 1187, 1510, and 1678 becomes more prominent. However, these fragments are fragments derived from a C region of an antibody and do not contribute to analysis of peptide fragments containing CDRs. Therefore, the protease digestion time was set to 6 hours, and the following examination was performed.

### [Experiment 4: Trypsin Digestion and Mass Spectrometry of Trastuzumab or Bevacizumab]

An antibody-immobilizing porous body was prepared using trastuzumab or bevacizumab instead of IgG as an antibody. Under the conditions selected in the experiment 2 and the experiment 3, that is, the conditions that the amount of the protease-immobilizing nanoparticles with respect to 100 µg of a solid phase antibody is 10 µg, and the digestion time is 6 hours, trypsin digestion was performed and mass spectrometry was performed. Based on the mass spectrometry results, database (Mascot server) analysis was performed. As a result, both trastuzumab and bevacizumab were identified with extremely high scores.

In the present experiment, more detailed mass spectrometry was performed in order to confirm that peptide fragments of trastuzumab or bevacizumab were detected by mass spectrometry.

Further, analysis of a supernatant after digestion was performed using LC-MS (Shimadzu Seisakusho, LCMS-8080 Triple-quadrupole ultra-high-performance liquid chromatography-MS). When the measurement using LC-MS was performed, a sample obtained by adding a formic acid to the supernatant such that a final concentration was 0.5% was used, and LC conditions were as follows.

### <HPLC Solution>

Solution A: 0.1% formic acid, 1% acetonitrile / aqueous solution
Solution B: 0.1% formic acid, acetonitrile solution

### <Column>

ShimPack ODS XR-ODS II (inner diameter: 2 mm; column length: 50 mm; Shimadzu GLC (Kyoto, Japan))
Column temperature: 40 °C
Flow rate: 0.4 mL/minute
Injection volume: 20 µL

### <Gradient Program>

0-2 minutes: %B = 0
2 - 10 minutes: %B = 0 - 40 gradient
10 - 11 minutes: %B = 40 - 98 gradient
11 - 13 minutes: %B = 98
13 - 13.5 minutes: %B = 98 - 0 gradient
13.5 - 15 minutes: %B = 0

In a heavy chain (VH) and a light chain (LH) of trastuzumab, the peptide sequences detected and identified by mass spectrometry are, in the case of trastuzumab, for example, IYPTNGYTR (SEQ ID No: 1; a portion of VH CDR2), FTISADTSK (SEQ ID No: 2; a portion of VH CDR3), ASQDVNTAVAWYQQKPGK (SEQ ID No: 3; a portion of VL CDR1), NTAYLQMNSLR (SEQ ID No: 4; a portion of VH CDR3), GLEWVAR (SEQ ID No: 5; a portion of VH CDR2), DTYIHWVR (SEQ ID No: 6; a portion of VH CDR1), and the like, and, in the case of bevacizumab, for example, STAYLQMNSLR (SEQ ID No: 7; a portion of VH CDR3), FTFSLDTSK (SEQ ID No: 8; a portion of VH CDR3), GLEWVGWINTYTGEPTYAADFKR (SEQ ID No: 9; a portion of VH CDR2, and CDR2), VLIYFTSSLHSGVPSR (SEQ ID No: 10; a portion of VL CDR2, CDR2, and a portion of CDR3), and the like. Since a sequence characteristic to each of amino acid sequences of trastuzumab and bevacizumab has been detected, these antibodies were actually identified

### [Experiment 5: Examination of Mixed Protease Digestion]

In the following, in order to examine applicability of mixed protease digestion in a system of the present invention, a protease digestion experiment was performed using a combined system of trypsin and lysyl endopeptidase (Lys-C).

Similar to the above experimental examples, an antibody-immobilizing porous body, in which 100 µg of an antibody (IgG or Herceptin) was immobilized on Protein G of the porous body, and nanoparticles on which 10 µg of a protease was immobilized, were stirred at 37 °C for 6 hours, and protease digestion of the antibody was performed. For each of the case where IgG was used as the antibody and the case where Herceptin was used the antibody, experiments were performed with a ratio (weight ratio) of trypsin to lysyl endopeptidase set to (1) 10:0, (2) 9:1, (3) 8:2, (4) 0:10, and immobilized components in a supernatant liquid and on a surface of a porous body after digestion were each analyzed using SDS-PAGE electrophoresis.

In the case where Herceptin was used the antibody, the supernatants after digestion of the levels 1 - 4 were subjected to mass spectrometry in the same manner as in the above experiment 2. Further, based on the mass spectrometry results, database analysis by the Mascot server (Matrix Science, USA) was performed in the same way as in the experiment 4.

At the levels 2 and 3 for which trypsin and lysyl endopeptidase were used in combination, as an analysis result, in addition to an antigen binding region of Herceptin, a constant region of a human-derived antibody was shown. Further, at the level 4 for which only lysyl endopeptidase was used, an analysis result showed that Herceptin was not included and only a constant region of the antibody was predominantly detected.

Based on electrophoresis, it is clear that, as a ratio of lysyl endopeptidase increases, the number of peptide fragments of a supernatant increases, a band area also tends to increase, and digestion efficiency and a peptide collection rate also increase. Further, based on mass spectrometry, as the ratio of lysyl endopeptidase increases, the number of kinds or an amount of detected peptide fragments increases. However, it is clear that, at the levels 2 - 4, newly detected peptide fragments are most derived from an Fc domain of Herceptin, and regioselectivity of the protease digestion (characteristic of selectively digesting an Fab region) is decreased.

From these results, it is clear that, as the amount of lysyl endopeptidase used increases, although the antibody digestion efficiency improves, the regioselectivity of the protease digestion decreases, and, as a production amount of peptide fragments of a constant region increases, since a relative production amount of peptide fragments of a V region decreases, accuracy of detection and identification of the antibody tends to decrease. Therefore, according to the method of the present invention, regioselective cleavage of an Fab region of an antibody was performed. In view of performing specific detection of CDRs, it is preferable to use trypsin alone. When trypsin and lysyl endopeptidase are used in combination, it is preferable to set a mixing amount of lysyl endopeptidase to 10% or less.

Further, at the level 4 for which only lysyl endopeptidase was used, site-specific digestion was not performed, whereas at the level 1 (experiment 4) for which only trypsin was used, a CDR is efficiently detected based on database analysis. Therefore, it is clear that a V region of an Fab region is selectively protease-digested. According to the above results, it is shown that, in the case where a substrate protein is the antibody, when the present invention is applied using trypsin, steric access of the protease to the antibody is appropriately controlled, and regioselective protease digestion is possible.

Further, the present experiment has shown that, when trypsin and lysyl endopeptidase are used in combination, each protease, without losing its function, can cleave an immobilized substrate protein in the pores. Since a V region of an antibody exists at front end of a molecule, when the amount of lysyl endopeptidase used increases, regiospecificity tends to decrease. However, it is suggested that, when other proteins are used as a substrate, it is possible that a combination system of proteases allows regioselectivity and digestion efficiency to be improved.

As described above in the experimental examples, it is clear that, according to the present invention, a protein such as an antibody can be regioselectively protease-digested using a simple method to obtain a peptide fragment sample, and the obtained peptide fragment sample is suitable for identification and quantification of the protein by mass spectrometry.

### [Experiment 6: Examination of Yield Increase of Peptide Fragment in Protease Digestion of Antibody]

In the following, conditions were examined that allow a yield of a peptide fragment to be increased when trastuzumab or bevacizumab, which is a pharmaceutical antibody, is trypsin-digested.

### <Experiment Procedures>

An experiment was conducted according to the following procedures.

### (Step 1)

An antibody drug (trastuzumab, bevacizumab) is placed in a low adsorption tube (Richel Micro Retico Tube 92017 (Richell (Toyama, Japan))) and is diluted with 180 µL of PBS. A concentration of the diluted solution was adjusted to 10 µg/mL, 3.3 µg/mL and 1.1 µg/mL in a 3-fold dilution series starting from 10 µg/mL. Each sample is analyzed at N = 6.

### (Step 2)

10 µL of antibody fractionation beads (Pierce 53126 Protein G UltraLink Resin (Pierce)) is dispensed into a filter tube (Millipore UFC30LG00 Ultrafree-C3LCR (Millipore) 0.2 µm).

### (Step 3)

The serum diluted sample of Step 1 is transferred to the filter tube of Step 2.

### (Step 4)

The mixture is stirred for 2 hours at room temperature while being lightly tapped using a tapping rotary mixer (NRC-20D model, Nissin Rika Corporation (Tokyo, Japan)).

### (Step 5)

Centrifuge filtration (10,000 x g, 1 minute) is performed and a solution is separated. 200 µL of PBS is added and centrifugal filtration is performed, and a filtrate is discarded. This operation is performed three times, and antibody fractionation beads (antibody amounts are respectively 1.0 µg, 0.33 µg, and 0.11 µg) are collected.

### (Step 6)

200 µL of an enzyme reaction buffer (25 mM Tris-HCl, pH 8.0) is added to the antibody fractionation beads of Step 5.

### (Step 7)

FG beads for antibody digestion (trypsin: 3 µg) are quickly uniformly dispersed on ice using an ultrasonic washer or a vortex mixer and are added to the enzyme reaction buffer of Step 6.

### (Step 8)

A solution collection tube is attached to the filter tube and is sealed using a parafilm or the like on a lid side. A protease digestion reaction of the antibody is performed by stirring at 37 °C for 6 hours while tapping with a tapping rotary mixer adjusted to a rotation speed of 1 - 5 rpm and a tapping speed of 2 times/minute. In this case, as a comparison group, stirring is performed using a vortex mixer.

### (Step 9)

The reaction mixture of Step 8 (10,000 x g, 1 minute) is centrifuged and filtered, resin is removed, and a filtrate is collected.

### (Step 10)

15 µL of enzyme reaction stop solution (10% formic acid) is added to the filtrate of Step 9.

### (Step 11)

The reaction stopped solution of Step 10 is transferred to an LCMS vial set (Shimadzu GLC GLC4010-VP Target vial VP, C4010- 630P Target PP Polyspring), and bubbles at a bottom are removed, and a sample for analysis is obtained.

### (Step 12)

The sample for analysis of Step 11 is set in an LCMS autosampler, and analysis is performed.

<Results>

From peptide sequences detected and identified by mass spectrometry, in the case of trastuzumab, IYPTNGYTR (SEQ ID No: 1; a portion of VH CDR2), FTISADTSK (SEQ ID No: 2; a portion of VH CDR3), ASQDVNTAVAWYQQKPGK (SEQ ID No: 3; a portion of VL CDR1), NTAYLQMNSLR (SEQ ID No: 4; a portion of VH CDR3), GLEWVAR (SEQ ID No: 5; a portion of VH CDR2), and DTYIHWVR (SEQ ID No: 6; a portion of VH CDR1) were selected; and, on the other hand, in the case of bevacizumab, STAYLQMNSLR (SEQ ID No: 7; a portion of VH CDR3), FTFSLDTSK (SEQ ID No: 8; a portion of VH CDR3), GLEWVGWINTYTGEPTYAADFKR (SEQ ID No: 9; a portion of VH CDR2, and CDR2), VLIYFTSSLHSGVPSR (SEQ ID No: 10; a portion of VL CDR2, CDR2, and a portion of CDR3) were selected, and yields of the peptide fragments when reaction occurred under tapping rotation stirring or vortex stirring were determined from LCMS data (MRM ionic strength). Significant differences of quantitative values were determined using T-test and were marked as p < 0.05 (*) and p < 0.01 (**).

The results for relative yields of peptide fragments including amino acid sequences of SEQ ID Nos: 1 - 6 were shown in Fig. 4; and the results for relative yields of peptide fragments including amino acid sequences of SEQ ID Nos: 7 - 8 were shown in Fig. 5.

From the figures, it is clear that, as the results of the protease digestion reactions of trastuzumab and bevacizumab, the yields of the peptide fragments, which are the products of the reactions, are significantly different depending on stirring methods (that is, tapping rotation stirring or vortex stirring), and the yields of the peptide fragments in the case of tapping rotation stirring tend to increase by about 3 - 5 times in the case of trastuzumab and about 1.4 - 4 times in the case of bevacizumab as compared to the case of vortex stirring (rotation speed: 100 rpm). Protease digestion can be caused to efficiently proceed by tapping rotation stirring (preferred rotation speed: 3 - 10 rpm; preferred tapping number: 1 - 5).

Further, from the figures, it is clear that, since a relative yield ratio of tapping rotation stirring to vortex stirring varies by varying a substrate:enzyme quantitative ratio, it is necessary to determine an optimal substrate:enzyme quantitative ratio in a range in which an antibody concentration is, for example, 1.1 µg/mL - 10 µg/mL (100 µL used per a reaction scale of 200 µL) such that an optimal peptide fragment yield is achieved.

Further, it is clear that, in the protease digestion reaction of the antibody at Step 8, by using a salt (NaCl)-free enzyme reaction buffer (preferred pH: 7.0 - 9.0; a pH of about 8.0 in the case of trypsin), aggregation of solid phase particles is prevented and good dispersion is obtained.

### Industrial Applicability

A protease decomposition reaction with a restricted reaction field has been known, However, substantially no attempt has been made to increase a yield of a peptide fragment (mainly, a peptide fragment containing a variable region or an Fab region, or a portion thereof, of a heavy chain or a light chain of an antibody), which is a decomposition product of an antibody, by increasing efficiency of a reaction between solid phases. However, according to the present invention, a yield of a peptide fragment of an antibody can be efficiently increased using a very simple method. As a result, accurate quantification of pharmaceutical antibodies in a biological sample such as blood using LCMS can be performed. Therefore, side effects can be reduced in patients at medical sites and effective treatments by doctors become possible.

SEQ ID Nos: 1 - 10: antibody fragments

All publications, patents and patent applications cited in the present specification are incorporated by reference in their entirety in the present specification.

## Claims

1. A method for obtaining, from a target monoclonal antibody, a peptide fragment comprising an Fab region or a variable region of the antibody or a portion of the region, the method comprising a process in which the target monoclonal antibody is digested with a protease by bringing the target monoclonal antibody, which is immobilized on inner surfaces of pores of a porous body, and the protease, which is immobilized on surfaces of nanoparticles having an average particle size larger than an average pore diameter of the porous body, into contact with each other in a liquid by stirring or tapping rotation stirring at a suitable speed so as to maintain a uniform dispersion.

2. A method for increasing a yield, from a target monoclonal antibody, of a peptide fragment that comprises an Fab region or a variable region of the antibody or a portion of the region, the method comprising a process in which the target monoclonal body is digested with a protease by bringing the target monoclonal antibody, which is immobilized on inner surfaces of pores of a porous body, and the protease, which is immobilized on surfaces of nanoparticles having an average particle size larger than an average pore diameter of the porous body, into contact with each other in a liquid by stirring or tapping rotation stirring at a low speed of less than 100 rpm so as to maintain a uniform dispersion, the average pore diameter of the porous body being in a range of 10 nm - 200 nm and the average particle size of the nanoparticles being in a range of 50 nm - 500 nm.

3. The method according to any one of claims 1 and 2, wherein the average particle size of the nanoparticles is 1.5 times to 4 times larger than the average pore diameter of the porous body.

4. The method according to any one of claims 1-3, wherein the stirring or the tapping rotation stirring is performed at a low speed of 50 rpm or less.

5. The method according to any one of claims 1-4, wherein the protease is selected from a group consisting of trypsin, Lys-C, Arg-C, Asp-N, chymotrypsin, V8 protease, and combinations of two or more thereof.

6. The method according to any one of claims 1-5, wherein the target monoclonal antibody is bound to the inner surfaces of the pores of the porous body via a linker molecule.

7. The method according to any one of claims 1-6, wherein the peptide fragment is a peptide fragment that comprises a complementarity-determining region (CDR) or a portion thereof.

8. The method according to any one of claims 1-6 further comprising a process of separating a target monoclonal antibody or an antibody population containing the target monoclonal antibody from a biological sample.

9. The method according to claim 8, wherein the biological sample is blood, serum, plasma, a tissue or a cell.

10. The method according to any one of claims 8 and 9 further comprising a process of binding the antibody population containing the target monoclonal antibody to the surfaces of the pores of the porous body.

11. The method according to any one of claims 1 - 10, wherein the liquid is a salt-free buffer solution.

12. The method according to any one of claims 1-11, wherein the target monoclonal antibody is an antibody therapeutic agent.

13. A kit for use in the method according to any one of claims 1 - 12, comprising: (i) a porous body having pores capable of immobilizing a target monoclonal antibody, (ii) nanoparticles immobilizing a protease on surfaces thereof, and (iii) an instruction manual for instructing a user to bring the target monoclonal antibody and the protease into contact with each other in a liquid by stirring or tapping rotation stirring at a low speed of less than 100 rpm so as to maintain a uniform dispersion, wherein an average particle size of the nanoparticles is larger than a pore diameter of the porous body.

14. The kit according to claim 13 further comprising an affinity means for separating and purifying an antibody.

15. The kit according to any one of claims 13 and 14, wherein the porous body has a linker molecule bound to the surfaces of the nanoparticles and inner surfaces of the pores.

16. The kit according to claim 15 wherein the linker molecule is an antibody binding polypeptide.
